# EUROPEAN PATENT APPLICATION

(11) **EP 4 434 558 A1**
(43) Date of publication of application: **25.09.2024**
(21) Application number: 24164407.9
(22) Date of filing: 19.03.2024
(51) Int. Cl.: A61M 5/145, A61M 5/172, H02P 6/20, A61M 5/142

(54) **SYSTEMS AND METHODS FOR DRIVING ELECTROMAGNETIC MOTORS OF MEDICAMENT INFUSION PUMPS**

(30) Priority: 23.03.2023 US 202363491836 P; 06.03.2024 US 202418596792
(71) Applicant: Medtronic MiniMed, Inc., Northridge, CA 91325 (US)
(72) Inventor: Smith, Roger E., Northridge, 91311 (US); Fatemeh, Delijani, Northridge, 91325 (US)
(74) Representative: Zimmermann & Partner Patentanwälte mbB

(57) **Abstract**

Systems and methods for driving electrical motors of medicament infusion pumps are disclosed. An infusion device for delivering a medicament to a body of a user can include a reservoir to contain medicament, a dispenser to cause medicament to be moved out of the reservoir, and a motor operably coupled to the dispenser. The motor can include a stator defining a central opening and a rotor removably disposed therein. A controller coupled to the stator is configured to (1) supply a first signal to the stator to cause the rotor to rotate in a first direction for a first rotational amount less than a full rotation; and (2) to then supply a second signal to the stator to cause the rotor to rotate in a second, opposite direction for a second rotational amount greater than the first, thereby causing medicament to be dispensed from the reservoir via the dispenser.

## Description

This application claims the benefit of U.S. Provisional Patent Application Serial No. 63/491,836, filed March 23, 2023, the entire content of which is incorporated herein by reference.

### TECHNICAL FIELD

The present technology relates generally to medical devices, and more particularly, to systems and methods for driving electromagnetic motors of medicament infusion pumps.

### BACKGROUND

Ambulatory infusion pumps are relatively small, at least substantially self-contained devices that are used to introduce drugs and other infusible substances (collectively "medicament") into users' bodies. Some infusion pumps are configured to be worn on a belt, carried in a clothing pocket, or the like. Other infusion pumps are configured to be adhered to skin in patch-like fashion. Infusion pumps are advantageous in that they may be used to, for example, subcutaneously introduce (or "infuse") medicament on an ongoing or even continuous basis outside of a clinical environment. Infusion pumps are also advantageous in that they greatly reduce the frequency of subcutaneous access events such as needle-based shots. One example of a medicament that may be introduced by an infusion pump is a liquid formulation of insulin. Other exemplary medicaments that may be introduced by an infusion pump include, but are not limited to, drugs that treat cancers and drugs that suppress the perception of pain.

Many conventional infusion pumps have improved user health and quality of life. Nevertheless, the present inventors have determined that conventional infusion pumps are susceptible to a wide range of improvements. By way of example, but not limitation, the present inventors have determined that it would be desirable to provide an infusion pump that is smaller, simpler, more power-efficient, more reliable, and less costly than conventional infusion pumps. Smaller pumps may require tighter tolerances to operate properly, and slight variations may cause issues with proper operation of a pump.

### SUMMARY

Generally, in some embodiments in accordance with the present technology, a method for driving a medicament infusion device via an electromagnetic motor can include supplying a first signal to a stator of the electromagnetic motor, thereby rotating a rotor of the electromagnetic motor from an initial position in a first direction for a first rotational amount less than a full rotation. The method can further include, after rotating the rotor of the electromagnetic motor in the first direction, supplying a second signal to the stator, thereby rotating the rotor in a second, opposite direction for a second rotational amount greater than the first rotational amount to drive medicament out of the infusion device.

In some embodiments, the method can additionally include obtaining, via one or more sensors of the device, a signal indicative of an amount of rotation of the rotor while supplying the first signal to the stator to rotate the rotor in the first direction. After obtaining the signal, the device ceases supplying the first signal to the stator, and initiates supplying the second signal to the stator. In some embodiments, wherein obtaining the signal indicative of an amount of rotation of the rotor includes measuring a rotational position of the rotor with a sensitivity of about 10, 9, 8, 7, 6, 5, 4, 3, 2, or 1 degrees or less. In some embodiments, obtaining the signal indicative of an amount of rotation comprises obtaining a rotational velocity of the rotor. Obtaining the signal indicative of an amount of rotation of the rotor may include measuring a time elapsed while supplying the first signal.

In some embodiments, the second signal is insufficient to overcome a cogging torque to rotate the rotor from the initial position in the second direction. Additionally or alternatively, the rotor can include a permanent magnet and the stator can include a plurality of coil windings disposed around the rotor. The rotor can have a centerpoint that is radially offset from a central position defined by the plurality of stator cores. In some embodiments, the rotor can have a centerpoint that is nearer to a first one of the stator cores than to a second one of the stator cores.

Generally, in some embodiments in accordance with the present technology, an infusion device for delivering a medicament to a body of a user can include a reservoir configured to contain medicament therein, a dispenser configured to cause medicament to be moved out of the reservoir, and an electromagnetic motor operably coupled to the dispenser. The motor can include a stator having a plurality of coil windings disposed around a central opening, and a rotor removably disposed within the central opening. The rotor can take the form of a rotatable permanent magnet. A controller coupled to the stator can be configured to (1) supply a first signal to the coil windings to cause the rotor to rotate in a first direction for a first rotational amount less than a full rotation; and (2) to then supply a second signal to the coil windings to cause the rotor to rotate in a second, opposite direction for a second rotational amount greater than the first rotational amount, such that rotation in the second direction causes medicament to be dispensed from the reservoir via the dispenser.

In some embodiments, the device includes one or more sensors configured to sense a rotational position of the rotor. The controller can be further configured to transition from supplying the first signal to supplying the second signal based on one or more sensor signals obtained via the one or more sensors. In some embodiments, the one or more sensor signals indicate an amount of rotation of the rotor. Additionally or alternatively, the one or more sensors can be configured to obtain the rotational position of the rotor with a sensitivity of about 10, 9, 8, 7, 6, 5, 4, 3, 2, or 1 degrees or less. Optionally, the one or more sensors are configured to obtain a rotational velocity of the rotor.

In some embodiments, the rotor has a centerpoint that is radially offset from a central position defined by the stator cores. Additionally or alternatively, the rotor centerpoint may be nearer to a first one of the plurality of stator cores than to a second one of the plurality of stator cores. In some implementations, the dispenser comprises a moveable plunger disposed within the reservoir, and rotation of the rotor in the second direction causes the plunger to move within the reservoir to dispense medicament.

Generally, in some embodiments in accordance with the present technology, a device includes a means for containing medicament, a means for dispensing medicament from the medicament-containing means, and a means for actuating the medicament-dispensing means. The actuating means can include a rotor operably coupled to the medicament-dispensing means. The actuator means can additionally be configured to rotate the rotor in a first direction for a first rotational amount less than a full rotation and to then rotate in a second, opposite direction for a second rotational amount greater than the first rotational amount, such that rotation in the second direction causes medicament to be dispensed from the medicament-containing means.

In some embodiments, the device additionally includes a means for sensing rotation of the rotor. The device can be configured to initiate rotation of the rotor in the second direction based on one or more signals from the rotation-sensing means. In various embodiments, the medicament-containing means can include a reservoir, the medicament-dispensing means can include a plunger, and the actuating means can include an electromagnetic motor. The motor can include a stator having a plurality of coil windings surrounding a central opening. The rotor can take the form of a rotatable permanent magnet removably disposed within the central opening.

Further disclosed herein are systems and methods for driving electrical motors of medicament infusion pumps. An infusion device for delivering a medicament to a body of a user can include a reservoir to contain medicament, a dispenser to cause medicament to be moved out of the reservoir, and a motor operably coupled to the dispenser. The motor can include a stator defining a central opening and a rotor removably disposed therein. A controller coupled to the stator is configured to (1) supply a first signal to the stator to cause the rotor to rotate in a first direction for a first rotational amount less than a full rotation; and (2) to then supply a second signal to the stator to cause the rotor to rotate in a second, opposite direction for a second rotational amount greater than the first, thereby causing medicament to be dispensed from the reservoir via the dispenser.

Other technical features may be readily apparent to one skilled in the art from the following figures, descriptions, and claims.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1A is a perspective view of an infusion device in accordance with several embodiments of the present technology.
FIG. 1B is a perspective view of the bottom of the infusion device of FIG. 1A.
FIG. 2A is a perspective view of a durable assembly of the infusion device of FIGS. 1A and 1B.
FIGS. 2B and 2C are perspective views of certain components of the durable assembly illustrated in FIG. 2A.
FIG. 3A is a perspective view of a disposable assembly of the infusion device of FIGS. 1A and 1B.
FIG. 3B is a perspective view of certain components of the disposable assembly illustrated in FIG. 3A.
FIG. 4A is a perspective view of a motor assembly of the infusion device shown in FIGS. 1A-3B.
FIG. 4B is a top plan view of the motor assembly shown in FIG. 4A.
FIG. 5 is a graph of motor torque at various rotational positions in accordance with embodiments of the present technology.

### DETAILED DESCRIPTION

### I. Overview

The present technology includes systems and methods for driving electromagnetic motors of medicament infusion devices configured to be adhered to the user's skin above the delivery site (sometimes referred to as "patch pumps"). The infusion devices include a reservoir configured to receive and contain a medicament, a motor, and an insertion assembly having a cannula that is to be operatively connected to the reservoir. After applying the device to the skin, the user activates the insertion assembly to insert the cannula subcutaneously, through which medicament can be delivered. The electromagnetic motor can take the form of a stator including a plurality of coil windings disposed around a central opening, and a rotor disposed (optionally removably disposed) within the central opening. The motor can be operably coupled to a plunger or other dispensing mechanism coupled to the reservoir, such that rotation of the rotor causes medicament to be dispensed from the reservoir, through the cannula, and into the user's body. While the present technology is well suited for patch pumps as described below, the technology may be applied to other small medicament pumps, such as smaller infusion pumps worn on a user's belt or clothing and connected to the user by, for example, an infusion set.

As described in greater detail below, many electrical motors exhibit cogging torque, which is the torque created by the magnetic attraction between magnet(s) of the rotor and magnetic components (e.g., an iron core) of the stator. Cogging torque is generally considered an undesirable feature of such motors, as the initial torque required to overcome the cogging torque when initially starting the motor can be higher than would otherwise be needed to run the motor during normal operation. This may require a higher-powered motor than would otherwise be necessary, which may increase the overall size and/or power consumption of the infusion device.

The problem of cogging torque can be exacerbated when the rotor is not perfectly centered with respect to the stator. In the case of a two-piece infusion device, the rotor may be contained in one component (e.g., a disposable assembly) that is removably mated with another component (e.g., a durable assembly) that contains the stator. While mating the two components together may desirably position a centerpoint of the rotor at precisely a centerpoint defined by the stator, in practice, due to manufacturing and assembly tolerances, the centerpoints may not be precisely aligned when the two components are mated together. This offset can increase the magnetic attraction between the rotor and one or two iron cores, and thus increase the cogging torque, thereby increasing the amount of torque output required of the motor to begin rotation from a stationary position. In some instances, such a radial offset may render the device inoperable, as the maximum torque supplied by the motor may be insufficient to overcome the cogging torque.

The present technology can address these and other problems associated with infusion devices powered by electromagnetic motors. For instance, consider an infusion device in which an electromagnetic motor includes a rotor that is operably coupled to a plunger such that rotation of the rotor in a clockwise direction causes the plunger to move within the reservoir to dispense medicament. To ameliorate the burden of cogging torque, the motor may first rotate the rotor in a counterclockwise direction for less than a full rotation, from an initial equilibrium position to a "windup" position having higher potential energy than the initial equilibrium position. Once the rotor is rotated to the windup position, the direction of rotation is reversed, and the rotor begins to rotate in the clockwise direction, through the initial equilibrium position and onward, optionally through multiple successive rotations of the rotor. By starting this clockwise rotation from the windup position (rather than from the initial equilibrium position), the kinetic energy gained while moving clockwise from the windup position to the equilibrium position effectively reduces the amount of torque required to be supplied by the stator to achieve full rotation of the rotor and escape the magnetic attraction that causes the cogging torque. Once the rotor's rotational velocity is high enough, the drag associated with the cogging torque is negligible, and the motor can be operated continuously as needed. This process may be repeated each time the motor is initiated while the rotor is in a standstill position. As a result of this approach, the peak torque requirements of the motor can be reduced and/or the alignment tolerances can be relaxed, thereby reducing the size of the motor and/or the power drain associated with running the motor.

### II. Example Infusion Devices with Electromagnetic Motors

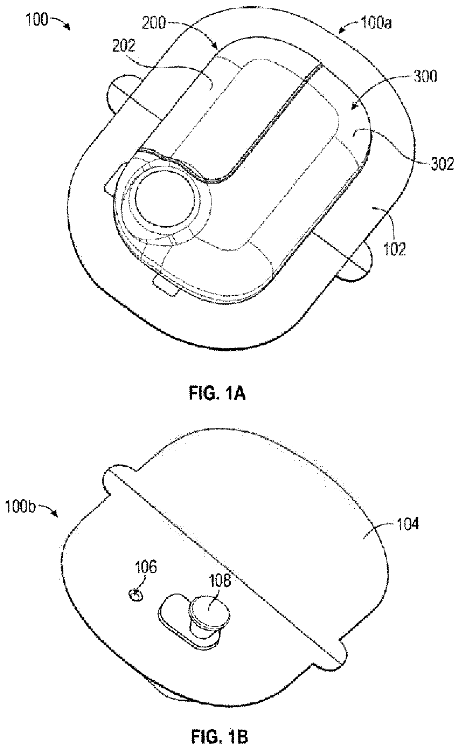

FIGS. 1A and 1B show top and bottom sides, respectively, of an infusion device 100 in accordance with several embodiments of the present technology. As previously mentioned, the bottom side 100b of the device 100 is configured to be adhered to the user's skin with the top side 100a facing away from the user. The device 100 includes a durable assembly 200 and a disposable assembly 300, each having respective housings 202 and 302. The durable assembly 200 and disposable assembly 300 are disposed on an adhesive pad 102 for securing to the user's skin, and an adhesive backing 104 to protect the adhesive pad 102 until the device 100 is secured to the user's skin. The bottom side 100b of the device 100 may also include a pull-before-use plug (PBUP) 108 and a fill port 106.

The device 100 may be used in conjunction with a wide variety of remote controllers (not shown). The remote controller, for example, can be a device-specific controller, a mobile phone, a tablet, etc. Such remote controllers may be used, for example, to enable the user to transmit instructions to the durable assembly 200 or otherwise facilitate communication between durable assembly 200 and the user (e.g., an alarm condition message or other message concerning the conditions of device 100). In some embodiments, the remote controller is configured to send instructions to and/or receive instructions from the disposable assembly 300.

With respect to dimensions, the device 100 can have a length of about 35-60 mm; a width of about 30-45 mm; and an overall thickness or height of about 8-18 mm. Suitable housing materials include, but are not limited to, plastic or other materials having a modulus of elasticity of 0.2-1.0 million psi.

To use the infusion device 100, the user (e.g., the patient) connects the disposable assembly 300 to the durable assembly 200. Unless the reservoir of the disposable assembly 300 has been sufficiently pre-loaded, the user injects a desired amount of medicament into the reservoir via the fill port 106. A plunger seek procedure (detailed below) may be initiated, either by the user or automatically. To adhere the device 100 to the user, the adhesive backing 104 is peeled off to expose the adhesive pad 102; the PBUP 108 may be removed; and/or the device 100 is positioned over the chosen body location and pressed gently to adhere the adhesive pad 102 to the skin surface. In some embodiments, the user triggers the automatic cannula insertion via the remote controller (e.g., after the plunger seek operation is complete). In some embodiments, plunger seek is not required.

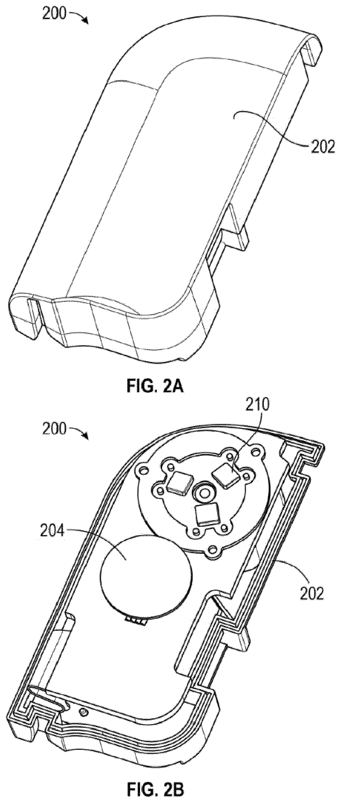

The durable assembly 200, shown in more detail in FIGS. 2A-2C, may include a housing 202, a buzzer or other alarm device 204, one or more batteries or other energy supply 206, a microprocessor (not shown), and a coil assembly 208 (which functions as a motor stator) including one or more Hall-effect sensors 210. In some embodiments, the energy supply 206 is a rechargeable battery, such as a rechargeable lithium battery, with enough power to drive the motor continuously without needing a capacitor or other additional energy storage device.

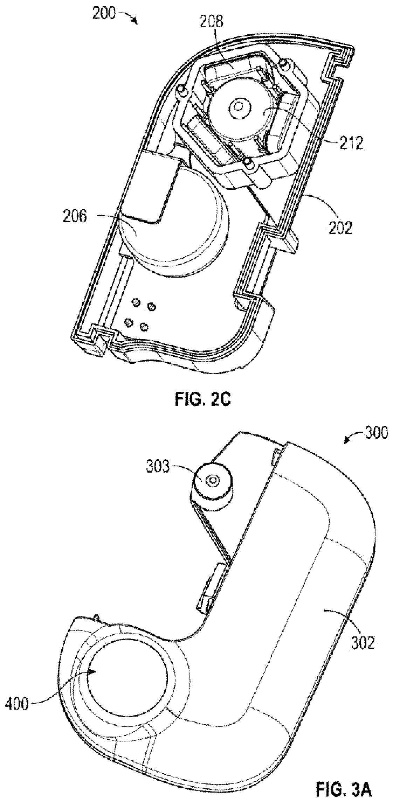

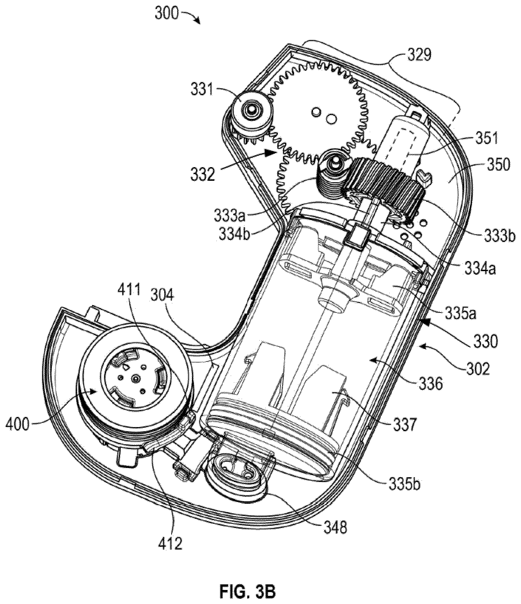

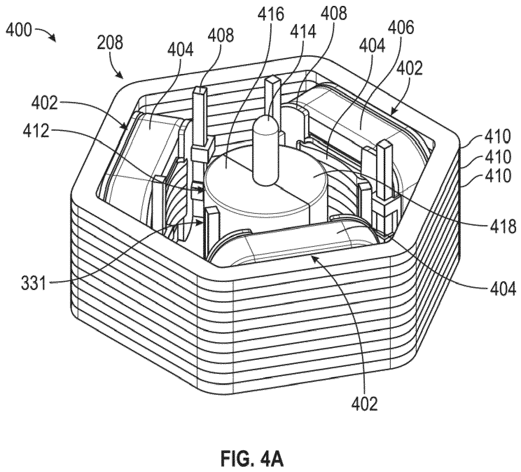

Referring specifically to FIG. 2C, the coil assembly 208 may be positioned around a recessed portion 212 of the durable assembly housing 202 that is configured to fit over a protruding portion 303 of the disposable housing 302 (FIG. 3A), which in turn fits over a magnetic motor rotor 331 of the disposable assembly 300 (FIG. 3B). In this two-piece motor, the motor's coil assembly 208 is in the durable assembly 200 and is positioned around the motor rotor 331 that is part of the disposable assembly 300. Together, the coil assembly 208 and the motor rotor 331 form an electromagnetic motor assembly, which is described in more detail below with respect to Figures 4A-5.

The disposable assembly 300, shown in more detail in FIGS. 3A and 3B, may include a reservoir assembly 330, a trigger assembly 304 (shown schematically), and an insertion assembly 400, all mounted on a baseplate 350. The reservoir assembly 330 may comprise a drive assembly 329, a reservoir 336, a plunger pusher 335a, and a plunger 335b. The plunger pusher 335a is coupled to the drive assembly 329, and both the plunger pusher 335a and the plunger 335b are contained within the reservoir 336.

Referring still to FIGS. 3A and 3B, the drive assembly may comprise the magnetic motor rotor 331 and a gear train 332. The gear train 332 is attached to the pusher 335a which is positioned in the reservoir 336. The magnetic motor rotor 331 may be mechanically attached through the gear train 332 to affect translation of the plunger pusher 335a (and the plunger 335b, when attached to the plunger pusher 335a) within the reservoir 336.

As best seen in FIG. 3B, the gear train 332 may include a worm drive comprised of a worm screw 333a and a worm gear 333b, and also a lead screw nut 334a and a fine-pitch lead screw 351 (enclosed by the lead screw nut 334a). The worm gear 333b is coupled to the lead screw 351 via the lead screw nut 334a. Protrusions 334b on the lead screw nut 334a correspond with recesses (not shown) inside the worm gear 333b, and a threaded portion (not shown) inside the lead screw nut 334a pairs with the thread on the lead screw 351 enclosed by the lead screw nut 334a. This configuration of the gear train 332 prevents back-driving due to reservoir pressure, eliminating the need for a clutch or other locking mechanism. Suitable materials for the components of the gear train 332 include, but are not limited to, stainless steel or high strength plastic, such as nylon, acetal (Delrin^{™}) or polycarbonate.

The reservoir 336 may be prefilled with a medicament. The medicament, for example, can be U-100 insulin or U-500 insulin or other concentrations of insulin to suit different user use profiles, or may be user-fillable by way of the fill port 106 (FIG. 1B). In some embodiments, the reservoir 336 can be mounted on a reservoir support block (not shown in FIG. 3B). A reservoir outlet fitting 348 is in fluid communication with the reservoir 336. The reservoir outlet fitting 348 is made from a drug-compatible material, such as, but not limited to, polypropylene, cyclic olefin polymer (COP) or polyethylene.

In those cases where the reservoir 336 is filled by the user, the user may completely fill the reservoir to capacity with medicament, or the user may choose to introduce less medicament and not completely fill the reservoir. Since an unknown amount of medicament may be injected into a user-filled reservoir, a plunger-pusher zeroing procedure (or "plunger seek") may be user-initiated or may be an automatic aspect of pump operation. A plunger seek procedure precisely determines and/or sets, before any medicament dispensing, exactly how far the plunger pusher 335a travels before it engages the plunger 335b, enabling a calculation to determine the amount of medicament in the reservoir and, therefore, an estimate of time-to-empty and time for disposable assembly replacement.

FIG. 3B shows the reservoir 336 before any medicament is introduced into the reservoir 336. The plunger 335b is disconnected from the plunger pusher 335a (and thus free-floating) and the plunger pusher 335a is in the fully retracted position. At this point, and until the plunger seek operation is complete, the PBUP 108 (FIG. 1B) may remain in place to prevent premature flow of medicament between the reservoir 336 and the insertion assembly 400. In other embodiments, a PBUP 108 may not be necessary. As medicament is introduced into the reservoir 336 via the fill port 106, the plunger 335b is pushed towards the plunger pusher 335a. If the reservoir 336 is filled to capacity, the plunger 336b will be pushed into contact with the plunger pusher 335a. In some embodiments, this causes the hooks 337 (or other suitable method of attachment) on the plunger 335b to engage with and permanently lock with the pusher 335a. If the reservoir 336 is not filled to capacity, the plunger 335b will be positioned at some unknown point within the reservoir 336 until the plunger seek operation is complete. Once the user has introduced medicament into the reservoir 336, a plunger seek operation can be initiated by the user or may be an automatic aspect of pump operation. When the plunger seek operation is initiated, the motor advances the plunger pusher 335a until it contacts the plunger 335b. In some embodiments, they lock together with plunger hooks 337 or some other suitable method of attachment. In some embodiments, the plunger pusher 335a and the plunger 335b are not configured to mechanically lock. The reservoir 336 and the plunger 335b may be made of cyclic olefin polymer (COP), polypropylene or other drug-compatible polymeric material. Suitable materials for the plunger pusher 335a include, but are not limited to, stainless steel, COP, nylon, and polycarbonate.

### III. Example Systems and Methods for Driving Electromagnetic Motors

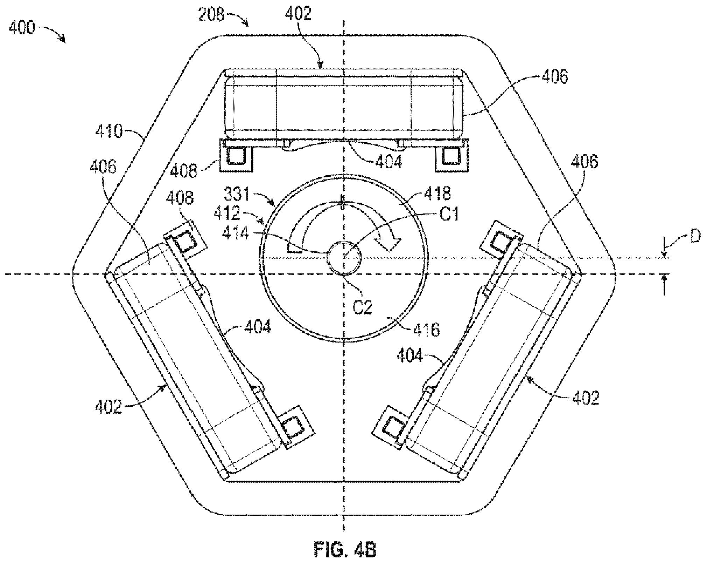

FIG. 4A is a perspective view of a motor assembly 400 of the infusion device shown in FIGS. 1A-3B, and FIG. 4B is a top plan view of the motor assembly 400 shown in FIG. 4A. The motor assembly 400 includes two components: the coil assembly 208, which functions as the stator and forms part of the durable assembly 200 (see FIG. 2C), and the motor rotor 331, which forms part of the disposable assembly 300 (see FIG. 3B). As noted previously, the coil assembly 208 can be positioned around a recessed portion 212 of the durable assembly housing 202 that is configured to fit over a protruding portion 303 of the disposable housing 302 (FIG. 3A), which in turn fits over the motor rotor 331 of the disposable assembly 300 (FIG. 3B). In this two-piece motor assembly 400, the motor's coil assembly 208 is in the durable assembly 200 and is positioned around the motor rotor 331 that is part of the disposable assembly 300. The housings 202 and 302 are omitted in FIGS. 4A and 4B for clarity.

The coil assembly 208 can include a plurality of sub-assemblies 402, each of which includes a stator core 404 surrounded by a coil winding 406. Although the illustrated example shows 3 sub-assemblies, in various examples there may be any suitable number of sub-assemblies, such as 2, 4, 5, 6, or more. Each sub-assembly 402 can include a frame assembly 408, which may include a bobbin, supports, etc. and may be made of a non-magnetic material and facilitate securing the sub-assemblies in place and coupling the coil windings 406 together.

In the illustrated example, a plurality of stator laminations 410 circumferentially define a perimeter of the coil assembly 208. These laminations include radially inward protrusions that define the stator cores 404 of the sub-assemblies 402. The stator laminations 410 and the cores 404 are made of iron or other suitable material, and may be configured to facilitate focusing magnetic flux generated by current passing through the coil windings 406.

The motor rotor 331 includes a permanent magnet 412 rotatably mounted over a rod 414 and coupled to the gear train 332 (see FIG. 3B). The permanent magnet 412 has a north pole 416 and a south pole 418, and the magnet 412 can be oriented such that when the magnet 412 rotates, the north pole 416 and the south pole 418 moves closer to and further from the various sub-assemblies 402 of the coil assembly 208. An exemplary magnet 412 of the motor rotor 331 may be a 2-pole, cylinder-shaped, rare earth (such as neodymium) rotor, magnetized across the diameter, with a 5 mm diameter and 5 mm height. Other suitable motor rotors may be larger or smaller, or be multi-pole. Motor rotors of this type typically cost about 5 cents per piece, helping control the total cost of disposable assembly 300.

In operation, when current is supplied to the coil assembly 208, the induced magnetic flux applies a torque to the motor rotor 331, causing the permanent magnet 412 of the motor rotor 331 to rotate. In particular, a microprocessor or other suitable controller (not shown) can direct rotation of the motor rotor 331 by sequentially energizing the coil windings 406 of the motor coil assembly 208 to create an electromagnetic torque coupling between the coil assembly 208 and the motor rotor 331. As noted previously, the motor rotor 331 is mechanically coupled to a gear train 332, which is attached to the pusher 335a and causes the pusher 335a to move within the reservoir 336 (see FIG. 3B). For instance, the rod 414 can be mounted to a first gear in the gear train 332 (see FIG. 3B), such that rotation of the rod 414 along with the magnet 412 causes the first gear in the gear train 332 to rotate. In other embodiments, a first gear in the gear train 332 may be mounted directly to the motor rotor 331 such that rotation of the motor rotor 331 causes the first gear in the gear train 332 to rotate.

In various embodiments, one or more sensors can be configured to detect and/or characterize movement of the motor rotor 331. For instance, the position/orientation of the motor rotor's 331 poles 416/418 relative to the rotating magnetic field generator (coil assembly 208) may be measured by back electromagnetic force (EMF), a rotary encoder(s), one or more Hall-effect sensors 210 (see FIG. 2B), or the like. For instance, the Hall-effect sensors 210 mounted adjacent to the coil windings 208 may be used to supply the microprocessor a count, a tachometer signal, or rotor position, enabling low-cost, closed-loop control of the motor rotor 331 position and speed. In some implementations, the sensor(s) may be configured to measure a rotational velocity of the motor rotor 331 with respect to the coil assembly 208. As described in more detail elsewhere herein, these sensor measurements can be used to efficiently drive the motor assembly 400 in a manner that overcomes a cogging torque during startup from an initial equilibrium position.

As noted above, the motor rotor 331 can be enclosed by a protruding portion 303 of the disposable assembly housing 302 (see FIG. 3A), while the coil assembly 208 is mated with the housing in a manner that provides a recessed portion 212 of the housing 202 (see FIG. 2C), which is configured to receive the protruding portion 303 and the motor rotor 331 therein. In this configuration, there is a gap between the motor coil assembly 208 and the motor rotor 331. Some or all of the gap may be defined by (and occupied by) housing portions, e.g., durable housing recessed portion 212 and disposable housing protruding portion 303 in the illustrated implementation. In other implementations, the gap between the motor coil assembly 208 and the motor rotor 331 may be occupied by only a portion of the durable assembly housing 202, or only a portion of the disposable assembly housing 302, or no structure at all and may simply be an air gap. The size of the gap, which is defined by the distance between the motor coil assembly 208 and the motor rotor 331, can be about 0.5 mm to 2.0 mm. As such, this enables no direct gear engagement or other mechanical connection between the durable assembly 200 and the disposable assembly 300. All electronics may be positioned within the durable assembly 200, with the energy needed by the disposable assembly 300 transferred by electromagnetic torque coupling, which is a coupling without direct mechanical coupling or electrical contact from the durable assembly 200. These designs afford the additional advantage of being relatively simple to make waterproof, or at least water resistant.

To ensure that the coil assembly 208 and the motor rotor 331 can be reliably mated together, the durable housing recessed portion 212 and the disposable housing protruding portion 303 can be manufactured with a minimum gap between a radially outer surface of the protruding portion 303 and the radially inner portion of the recessed portion 212 (see FIGS. 2C and 3A). This gap, however, can result in a misalignment between the motor rotor 331 and the coil assembly 208. In ideal operating conditions, a radial centerpoint C1 of the motor rotor 331 (e.g., a centerpoint of the rod 414 about which the magnet 412 rotates) is aligned exactly with a centerpoint C2 of the coil assembly 208 (e.g., a centerpoint that is equidistant from each of the stator cores 404 of the sub-assemblies 402). In the illustrated example shown in FIG. 4B, the centerpoint C1 of the motor rotor 331 is offset from the centerpoint C2 of the coil assembly 208 by a distance D. In various examples, the distance D may be, for example, about 10% or more of the diameter of the magnet 412 of the motor rotor 331 (e.g., for a magnet 412 having a diameter of 5 mm, the offset distance D may be about 0.5 mm). In various examples, the offset distance D may be at least about 5%, 10%, 15%, or 20% of the diameter of the motor rotor 331.

In a perfectly aligned motor (e.g., offset distance D of 0), rotating the motor rotor 331 by externally turning the rod 414 (i.e., not driving the motor electrically) will result in a variable torque required to overcome the magnetic attraction between the rotor poles 416, 418 and the stator cores 404. This can be referred to as cogging torque. In this example, there would typically be six cogging positions requiring increased torque per revolution, corresponding to magnetic attraction between each of the two rotor poles 416, 418 and the three stator cores 404. These cogging positions can be represented as torque wells, also referred to herein as magnetic detents, which are rotational positions to which the motor rotor 331 is drawn due to the magnetic attraction between the rotor poles 416, 418 and the stator cores 404. In practice, this cogging torque must be overcome by the motor output torque being high enough for the motor to start and run. Cogging torque may generally be minimized by motor manufacturers through design. For example, under ideal conditions (including perfect alignment of the motor rotor 331 and the coil assembly 208), the cogging torque may have a magnitude of only a few percent of the maximum rotor torque.

As the offset distance D increases, however, the cogging torque grows larger. In the example shown in FIG. 4B, the relatively weak, six-position cogging present in the ideal aligned case instead becomes two strong cogging positions. These positions correspond to each of the two rotor poles 416, 418 aligning with the nearest stator core 404 (i.e., the uppermost sub-assembly 402 shown in FIG. 4B). The magnitude of the cogging torque may be similar to or even greater than the maximum rotor torque, depending on the exact offset. In practice, even slight rotor offsets can pose risks to reliable motor starting.

While increased cogging torque may be addressed by increasing the maximum rotor torque, this approach can lead to increased cost, complexity, and power consumption of the motor assembly 400. Accordingly, the present technology provides systems and methods for operating the motor assembly 400 in a manner that overcomes the cogging torque by taking advantage of increasing potential energy as the motor rotor 331 moves away from a cogging position to create additional kinetic energy in the desired direction of rotation.

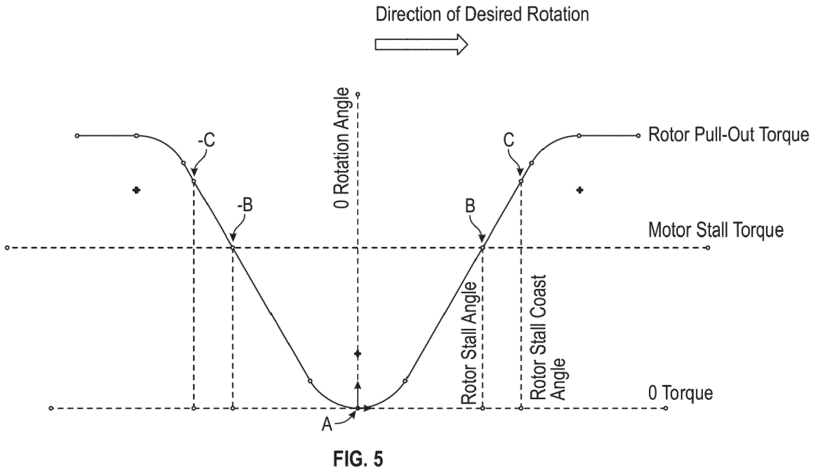

This technique is best illustrated in FIG. 5, which depicts a graph of motor torque at various rotational positions in accordance with embodiments of the present technology. The horizontal axis depicts the rotational position of the motor rotor 331, with the positive x-axis indicating a desired direction of rotation (e.g., movement in a direction to effect movement of the plunger to dispense medicament from the reservoir). In the following discussion, the desired direction of rotation is clockwise; however, depending on the particular configuration of the device, the desired direction of rotation may instead be counterclockwise. The vertical axis depicts the torque required to move the motor rotor 331 to various rotational positions, with the cogging position indicated by the lowest point (and 0 rotation angle), labeled A. This is the initial equilibrium position, to which the motor rotor 331 will return when no external torque is applied to the motor rotor 331 (although a single equilibrium position is shown, as noted above there may be two or more equilibrium (or cogging) positions depending on the arrangement of the motor assembly. As described herein, external torque can be supplied by applying a drive signal (e.g., a drive waveform) to the motor assembly 400 to cause the motor rotor 331 to rotate in one direction or another depending on the polarity of the drive signal.

As shown in FIG. 5, the shape of the torque curve is variable but generally roughly symmetrical about the vertical line corresponding to 0 rotation angle. This reflects the fact that it takes torque to move the motor rotor 331 either way (clockwise or counterclockwise) away from the cogging position. Accordingly, the cogging position may be thought of as either a magnetic detent or a torque well. During operation, the motor rotor 331 naturally stops at the magnetic detent position due to the high pull-out torque required. In other words, the motor rotor 331 nearly always starts from position A shown in FIG. 5.

As shown, there are five positions identified on FIG. 5 in addition to several torques and positions. Position A is the minimum energy position corresponding to the actual assumed stop position of the un-energized motor rotor 331. As this is the bottom of the torque well, energy is required to move the motor rotor 331 from this magnetic detent. Point B indicates a rotor stall angle, which is the equilibrium point between torque supplied when maximum current is applied to the motor and the cogging torque urging the motor rotor 331 back toward the initial position A. In operation, however, when maximum current is supplied to the motor starting from an initial stationary state at position A, the motor rotor 331 first accelerates to the right along the torque curve (low detent torque). This causes the motor rotor 331 to acquire kinetic energy, enabling it to overshoot position B and finally come to a standstill (0 rotational velocity) at position C. From this position, however, the torque from supplying maximum current to the motor is insufficient to reach the rotor pull-out torque, and as such the motor rotor 331 "slides back" to the static torque position B (i.e., the motor rotor 331 rotates in the counterclockwise direction), until it reaches the static equilibrium at position B. In other words, the torque generated from the maximum current to the motor is equal to the pullback force of the magnetic forces in the magnetic detent, thereby stalling the motor rotor 331 at position B.

Also shown in FIG. 5 are the corresponding counterclockwise rotation positions labelled B- and C-. These are the equivalent positions to B and C when the motor is driven in the opposite position. In other words, supplying maximum current of opposite polarity to rotate the motor rotor 331 in the counterclockwise direction will result in the motor rotor 331 reaching a maximum rotation at position C- before rotating clockwise to a static state at position B-.

As seen in FIG. 5, simply driving the motor clockwise with maximum torque is not sufficient to cause the motor rotor 331 to pull out of the magnetic detent position and start continuous rotation, even with the coasting effect considered (i.e., coasting due to acquired kinetic energy moves the motor rotor 331 only to position C, after which it slides back to position B). However, this same dynamic can be utilized to overcome the cogging torque without requiring higher maximum torque supplied by the motor.

In accordance with some embodiments of the present technology, to overcome the cogging torque, the motor may first rotate the motor rotor 331 in a first direction for less than a full rotation, from an initial equilibrium position to a "windup" position having higher potential energy than the initial equilibrium position, and then rotate the motor rotor 331 in the opposite direction, through the initial equilibrium position, and optionally to one or more full rotations of the motor rotor 331.

In the example shown in FIG. 5, if maximum current is applied to rotate the motor rotor 331 in the counterclockwise direction, this windup position corresponds to position C- (or B-if enough time passes for the motor rotor 331 to slide back to the equilibrium at position B-). Once the motor rotor 331 is rotated to the windup position, the current supplied to the motor is reversed (e.g., delivering maximum current of the opposite polarity), thereby causing the direction of motor rotation to reverse. As the motor rotor 331 begins to rotate in the clockwise direction, the motor rotor 331 moves through the initial equilibrium position A and continues rotating clockwise. However, because the motor rotor 331 now has additional kinetic energy due to its rotational movement from the windup position C- or B- to position A, the torque supplied by the motor is sufficient to achieve the rotor pull-out torque required to escape the magnetic detent. In other words, by starting the clockwise rotation from the windup position C- or B- (rather than from the initial equilibrium position A), the kinetic energy gained while moving clockwise from the windup position to the equilibrium position effectively reduces the amount of torque required to be supplied by the stator to achieve full rotation of the motor rotor 331. Once the motor rotor's rotational velocity is sufficiently high, the drag associated with the cogging torque is negligible, and the motor can be operated continuously as needed. This process may be repeated each time the motor is initiated while the motor rotor 331 is in a standstill position. As a result of this approach, the peak torque requirements of the motor can be reduced, thereby reducing the size of the motor and/or the power drain associated with running the motor.

In the illustrated example, the potential energy stored in the magnetic field at B- is turned into kinetic energy as the motor rotor 331 crosses back through A, as well as increasing the angle through which energy can be supplied to drive the motor rotor 331 to position B. This theoretically more than doubles the available energy to drive the motor rotor 331 past position B and position C. This "slingshot" approach takes advantage of the increased potential energy in moving the motor rotor 331 in an opposite rotational direction to achieve the pull-out torque without requiring higher total torque supplied by the motor alone. This effect can be conceptualized as similar to the process of "rocking" to get a car tire out of a hole. The car can be driven in a reverse for a short distance while the tire moves up one side of the hole without exiting the hole. The car is then driven forward, and the additional kinetic energy from the tire moving down the near side of the hole aids the tire in moving up the opposite side of the hole, which can help enable the tire to fully escape the hole.

While the example above describes rotating the motor rotor 331 to position B- or C-, in various examples the motor rotor 331 may be rotated to other rotational positions in the first direction, including rotation of the motor rotor 331 to positions that are not as fully rotated as positions B- or C-. Any initial amount of rotation in the counterclockwise direction can increase the kinetic energy of the motor rotor 331 as it moves in the clockwise direction, and as such in some instances it may not be necessary to achieve a "windup" position corresponding to position B- or C- in order to escape the magnetic detent. Similarly, while some examples described herein refer to supplying maximum current in one or the other direction, in various implementations the current levels may vary between rotational directions and/or may vary over time as desired.

In various examples, the rotational position and/or velocity of the motor rotor 331 can be measured, and the controller (e.g., a microprocessor or other suitable component) can use the sensor data to determine when to transition from rotating the motor rotor 331 in a first rotational direction to rotating the motor rotor 331 in the opposite rotational direction. For instance, the controller may supply current to the coil assembly to cause the motor rotor 331 to rotate counterclockwise until the motor rotor 331 achieves a predetermined rotational position (e.g., about 5, 10, 15, 20, 25, or 30 degrees away from position A in the counterclockwise direction). Once sensor data indicates this predetermined rotational position has been achieved, the controller can transition to supplying opposite polarity current to the coil assembly to drive the motor rotor 331 in the clockwise direction. In various examples, the predetermined rotational position may correspond to position C-, B-, or other suitable position along the torque curve illustrated in FIG. 5.

In some embodiments, rotational position can be obtained using combined readings from a plurality of analog Hall-effect sensors. Such analog Hall-effect sensors can be much more accurate than standard digital rotor position sensors frequently employed on brushless motors. While digital sensors accurately sense coarse rotor rotation (a typical system will use digital Hall-effect sensors to detect 60 degree rotation for coil commutation), analog Hall-effect sensors can resolve rotations of 1-2 degrees. With this rotor rotation angle resolution, the rotational position of the motor rotor 331 can be accurately determined and used for precise control of the motor, as is known in the art.

Additionally or alternatively to measuring rotational position, the controller may respond to sensor signals indicating that rotational velocity has fallen below a predetermined threshold (or even reached a rotational velocity of 0, indicating that the motor rotor 331 has reached position C- or has settled at position B-). In some examples, rotational velocity can be obtained by measuring the time between measured motor rotor 331 positions as detected via one or more analog Hall-effect position sensors. Additionally or alternatively, sensors that directly measure rotational velocity of the motor rotor 331 (e.g., back electromagnetic force (EMF), etc.) can be used. Once the rotational velocity has fallen below the predetermined threshold, the controller can supply a signal to the coil assembly to rotate the motor rotor 331 in the opposite direction.

In some implementations, the controller may utilize time-based signals to determine when to cease driving the motor rotor 331 in a first rotational direction and to initiate driving the motor rotor 331 in the opposite direction. For instance, upon startup from an initial static state (e.g., position A in FIG. 5), the controller may supply current to drive the motor rotor 331 in the counterclockwise direction for a predetermined period of time (e.g., a period of time that corresponds to the time it takes to move from position A to position B- or to position C- or other suitable position on the torque curve). After the predetermined period of time, the controller may switch to supplying current to drive the motor rotor 331 in the clockwise direction. In some embodiments, various sensor modalities can be combined for use in controlling the motor, including one or more of: rotational position sensing, torque sensing, total energy sensing, rotational velocity, time, etc. Similarly, the motor rotor 331 can be driven with a predetermined amount of energy to drive the motor rotor 331 in a first direction, and then the direction can be reversed.

### IV. Conclusion

Although the devices and methods are described in the context of medicament infusion devices such as patch pumps, it should be appreciated that the techniques are equally applicable to a variety of medical devices and/or non-medical devices powered by electromagnetic rotors, particularly devices having a small form factor and in which the motors exhibit cogging torque. It should also be noted here that the specification describes structures and methods that are especially well-suited for the subcutaneous delivery of high concentration insulin (i.e., U-200 insulin and above) such as U-500 insulin as well as lower concentration insulin such as U-100 insulin. Nevertheless, it should be appreciated that the present inventions are applicable to a wide variety of infusion pumps and medicaments. For example, the present inventions are also applicable to medicaments such as, for example, drugs to mask pain, chemotherapy and other cancer related drugs, antibiotics, hormones, GLP-1, glucagon, various other drugs that include large molecules and proteins that may require a high level of delivery accuracy, as well as to relatively high concentration insulin (i.e., U-200 insulin and above) such as U-500 insulin, as well as lower concentration insulin, such as U-100 insulin.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

The following examples are a non-limiting list of clauses in accordance with one or more techniques of this disclosure.

Example 1. A method for driving a medicament infusion device via an electromagnetic motor, the method comprising: supplying a first signal to a stator of the electromagnetic motor, thereby rotating a rotor of the electromagnetic motor from an initial static position in a first direction for a first rotational amount less than a full rotation to a windup position; and after rotating the rotor of the electromagnetic motor in the first direction to the windup position, supplying a second signal to the stator, thereby rotating the rotor from the windup position in a second, opposite direction for a second rotational amount greater than the first rotational amount to drive medicament out of the infusion device.

Example 2. The method of Example 1, further comprising: obtaining, via one or more sensors of the device, a signal indicative of an amount of rotation of the rotor while supplying the first signal to the stator to rotate the rotor in the first direction; and after obtaining the signal, ceasing supplying the first signal to the stator and initiating supplying the second signal to the stator.

Example 3. The method of Example 2, wherein obtaining the signal indicative of an amount of rotation of the rotor comprises measuring a rotational position of the rotor with a sensitivity of about 10 degrees or less.

Example 4. The method of any one of Example 2 or 3, wherein obtaining the signal indicative of an amount of rotation comprises obtaining a rotational velocity of the rotor.

Example 5. The method of any one of the preceding Examples, wherein obtaining the signal indicative of an amount of rotation of the rotor comprises measuring a time elapsed while supplying the first signal.

Example 6. The method of any one of the preceding Examples, wherein the second signal is insufficient to overcome a cogging torque to rotate the rotor from the initial position in the second direction.

Example 7. The method of any one of the preceding Examples, wherein the rotor comprises a permanent magnet and the stator comprises a plurality of coil windings each surrounding a stator core and disposed around the rotor, and wherein the rotor has a centerpoint that is radially offset from a central position defined by the stator cores.

Example 8. The method of any one of the preceding Examples, wherein the rotor comprises a permanent magnet and the stator comprises a plurality of coil windings each surrounding a stator core, and wherein the rotor has a centerpoint that is nearer to a first one of the stator cores than to a second one of the stator cores.

Example 9. An infusion device for delivering a medicament to a body of a user, the device comprising: a reservoir configured to contain medicament therein; a dispenser configured to cause medicament to be moved out of the reservoir; and an electromagnetic motor operably coupled to the dispenser, the motor comprising: a stator comprising a plurality of coil windings disposed around a central opening; and a rotor removably disposed within the central opening, the rotor comprising a rotatable permanent magnet; a controller coupled to the stator and configured to (1) supply a first signal to the coil windings to cause the rotor to rotate in a first direction for a first rotational amount less than a full rotation; and (2) to then supply a second signal to the coil windings to cause the rotor to rotate in a second, opposite direction for a second rotational amount greater than the first rotational amount, wherein rotation in the second direction causes medicament to be dispensed from the reservoir via the dispenser.

Example 10. The infusion device of Example 9, further comprising one or more sensors configured to sense a rotational position of the rotor.

Example 11. The infusion device of Example 10, wherein the controller is further configured to transition from supplying the first signal to supplying the second signal based on one or more sensor signals obtained via the one or more sensors.

Example 12. The infusion device of Example 11, wherein the one or more sensor signals indicate an amount of rotation of the rotor.

Example 13. The infusion device of any one of Examples 10-12, wherein the one or more sensors are configured to detect the rotational position of the rotor with a sensitivity of about 10 degrees or less.

Example 14. The infusion device of any one of Examples 9-13, further comprising one or more sensors configured to obtain a rotational velocity of the rotor.

Example 15. The infusion device of any one of Examples 9-14, wherein the rotor has a centerpoint that is radially offset from a central position defined by the stator.

Example 16. The infusion device of any one of Examples 9-15, wherein the rotor has a centerpoint that is nearer to a first one of the plurality of coil windings than to a second one of the plurality of coil windings.

Example 17. The infusion device of any one of Examples 9-16, wherein the dispenser comprises a moveable plunger disposed within the reservoir, and wherein rotation of the rotor in the second direction causes the plunger to move within the reservoir to dispense medicament.

Example 18. A device comprising: a means for containing medicament; a means for dispensing medicament from the medicament-containing means; and a means for actuating the medicament-dispensing means, the actuating means comprising a rotor operably coupled to the medicament-dispensing means and configured to rotate the rotor in a first direction for a first rotational amount less than a full rotation and to then rotate in a second, opposite direction for a second rotational amount more than the first rotational amount, wherein rotation in the second direction causes medicament to be dispensed from the medicament-containing means.

Example 19. The device of Example 18, further comprising a means for sensing rotation of the rotor, wherein the device is configured to initiate rotation of the rotor in the second direction based on one or more signals from the rotation-sensing means.

Example 20. The device of Example 18 or Example 19, wherein the medicament-containing means comprises a reservoir.

Example 21, The device of any one of Examples 18-20, wherein the medicament-dispensing means comprises a plunger.

Example 22. The device of any one of Examples 18-21, wherein the actuating means comprises an electromagnetic motor comprising a stator having a plurality of coil windings surrounding a central opening, the rotor comprising a permanent magnet removably disposed within the central opening.

Further disclosed herein is the subject-matter of the following clauses:
1. A method for driving a medicament infusion device via an electromagnetic motor, the method comprising:
   supplying a first signal to a stator of the electromagnetic motor, thereby rotating a rotor of the electromagnetic motor from an initial static position in a first direction for a first rotational amount less than a full rotation to a windup position; and
   after rotating the rotor of the electromagnetic motor in the first direction to the windup position, supplying a second signal to the stator, thereby rotating the rotor from the windup position in a second, opposite direction for a second rotational amount greater than the first rotational amount to drive medicament out of the infusion device.
2. The method of clause 1, further comprising:
   obtaining, via one or more sensors of the device, a signal indicative of an amount of rotation of the rotor while supplying the first signal to the stator to rotate the rotor in the first direction; and
   after obtaining the signal, ceasing supplying the first signal to the stator and initiating supplying the second signal to the stator.
3. The method of clause 1 or 2, wherein obtaining the signal indicative of an amount of rotation of the rotor comprises measuring a rotational position of the rotor with a sensitivity of about 10 degrees or less.
4. The method of clause 2 or of any of the clauses 1 to 3, wherein obtaining the signal indicative of an amount of rotation comprises obtaining a rotational velocity of the rotor.
5. The method of clause 2 or of any of the clauses 1 to 4, wherein obtaining the signal indicative of an amount of rotation of the rotor comprises measuring a time elapsed while supplying the first signal.
6. The method of clause 1 or of any of the clauses 1 to 5, wherein the second signal is insufficient to overcome a cogging torque to rotate the rotor from the initial position in the second direction.
7. The method of clause 1 or of any of the clauses 1 to 6, wherein the rotor comprises a permanent magnet and the stator comprises a plurality of coil windings each surrounding a stator core and disposed around the rotor, and wherein the rotor has a centerpoint that is radially offset from a central position defined by the stator cores.
8. The method of clause 1 or of any of the clauses 1 to 7, wherein the rotor comprises a permanent magnet and the stator comprises a plurality of coil windings each surrounding a stator core, and wherein the rotor has a centerpoint that is nearer to a first one of the stator cores than to a second one of the stator cores.
9. An infusion device for delivering a medicament to a body of a user, the device comprising:
   a reservoir configured to contain medicament therein;
   a dispenser configured to cause medicament to be moved out of the reservoir; and
   an electromagnetic motor operably coupled to the dispenser, the motor comprising:
      a stator comprising a plurality of coil windings disposed around a central opening; and
      a rotor removably disposed within the central opening, the rotor comprising a rotatable permanent magnet;
      a controller coupled to the stator and configured to (1) supply a first signal to the coil windings to cause the rotor to rotate in a first direction for a first rotational amount less than a full rotation; and (2) to then supply a second signal to the coil windings to cause the rotor to rotate in a second, opposite direction for a second rotational amount greater than the first rotational amount, wherein rotation in the second direction causes medicament to be dispensed from the reservoir via the dispenser.
10. The infusion device of clause 9, further comprising one or more sensors configured to sense a rotational position of the rotor.
11. The infusion device of clause 9 or 10, wherein the controller is further configured to transition from supplying the first signal to supplying the second signal based on one or more sensor signals obtained via the one or more sensors.
12. The infusion device of clause 11 or of any of the clauses 9 to 11, wherein the one or more sensor signals indicate an amount of rotation of the rotor.
13. The infusion device of clause 10 or of any of the clauses 9 to 12, wherein the one or more sensors are configured to detect the rotational position of the rotor with a sensitivity of about 10 degrees or less.
14. The infusion device of clause 9 or of any of the clauses 9 to 13, further comprising one or more sensors configured to obtain a rotational velocity of the rotor.
15. The infusion device of clause 9 or of any of the clauses 9 to 14, wherein the rotor has a centerpoint that is radially offset from a central position defined by the stator.
16. The infusion device of clause 9 or of any of the clauses 9 to 15, wherein the rotor has a centerpoint that is nearer to a first one of the plurality of coil windings than to a second one of the plurality of coil windings.
17. The infusion device of clause 9 or of any of the clauses 9 to 16, wherein the dispenser comprises a moveable plunger disposed within the reservoir, and wherein rotation of the rotor in the second direction causes the plunger to move within the reservoir to dispense medicament.
18. A device comprising:
   a means for containing medicament;
   a means for dispensing medicament from the medicament-containing means; and
   a means for actuating the medicament-dispensing means, the actuating means comprising a rotor operably coupled to the medicament-dispensing means and configured to rotate the rotor in a first direction for a first rotational amount less than a full
      rotation and to then rotate in a second, opposite direction for a second rotational amount more than the first rotational amount, wherein rotation in the second direction causes medicament to be dispensed from the medicament-containing means.
19. The device of clause 18, further comprising a means for sensing rotation of the rotor, wherein the device is configured to initiate rotation of the rotor in the second direction based on one or more signals from the rotation-sensing means.
20. The device of clause 18 or 19, wherein the medicament-containing means comprises a reservoir.
21. The device of clause 18 or of any of the clauses 18 to 20, wherein the medicament-dispensing means comprises a plunger.
22. The device of clause 18 or of any of the clauses 18 to 21, wherein the actuating means comprises an electromagnetic motor comprising a stator having a plurality of coil windings surrounding a central opening, the rotor comprising a permanent magnet removably disposed within the central opening.

## Claims

1. A method for driving a medicament infusion device via an electromagnetic motor, the method comprising:
supplying a first signal to a stator of the electromagnetic motor, thereby rotating a rotor of the electromagnetic motor from an initial static position in a first direction for a first rotational amount less than a full rotation to a windup position; and
after rotating the rotor of the electromagnetic motor in the first direction to the windup position, supplying a second signal to the stator, thereby rotating the rotor from the windup position in a second, opposite direction for a second rotational amount greater than the first rotational amount to drive medicament out of the infusion device.

2. The method of claim 1, further comprising:
obtaining, via one or more sensors of the device, a signal indicative of an amount of rotation of the rotor while supplying the first signal to the stator to rotate the rotor in the first direction; and
after obtaining the signal, ceasing supplying the first signal to the stator and initiating supplying the second signal to the stator.

3. The method of claim 2, wherein obtaining the signal indicative of an amount of rotation of the rotor comprises measuring a rotational position of the rotor with a sensitivity of about 10 degrees or less, and/or
wherein obtaining the signal indicative of an amount of rotation comprises obtaining a rotational velocity of the rotor, and/or
wherein obtaining the signal indicative of an amount of rotation of the rotor comprises measuring a time elapsed while supplying the first signal.

4. The method of any of the claims 1 to 3, wherein the second signal is insufficient to overcome a cogging torque to rotate the rotor from the initial position in the second direction.

5. The method of any of the claims 1 to 4, wherein the rotor comprises a permanent magnet and the stator comprises a plurality of coil windings each surrounding a stator core and disposed around the rotor, and wherein the rotor has a centerpoint that is radially offset from a central position defined by the stator cores, and/or
wherein the rotor comprises a permanent magnet and the stator comprises a plurality of coil windings each surrounding a stator core, and wherein the rotor has a centerpoint that is nearer to a first one of the stator cores than to a second one of the stator cores.

6. An infusion device for delivering a medicament to a body of a user, the device comprising:
a reservoir configured to contain medicament therein;
a dispenser configured to cause medicament to be moved out of the reservoir; and
an electromagnetic motor operably coupled to the dispenser, the motor comprising:
a stator comprising a plurality of coil windings disposed around a central opening; and
a rotor removably disposed within the central opening, the rotor comprising a rotatable permanent magnet;
a controller coupled to the stator and configured to (1) supply a first signal to the coil windings to cause the rotor to rotate in a first direction for a first rotational amount less than a full rotation; and (2) to then supply a second signal to the coil windings to cause the rotor to rotate in a second, opposite direction for a second rotational amount greater than the first rotational amount, wherein rotation in the second direction causes medicament to be dispensed from the reservoir via the dispenser.

7. The infusion device of claim 6, further comprising one or more sensors configured to sense a rotational position of the rotor.

8. The infusion device of claim 7, wherein the controller is further configured to transition from supplying the first signal to supplying the second signal based on one or more sensor signals obtained via the one or more sensors, particularly
wherein the one or more sensor signals indicate an amount of rotation of the rotor.

9. The infusion device of claim 7 or 8, wherein the one or more sensors are configured to detect the rotational position of the rotor with a sensitivity of about 10 degrees or less.

10. The infusion device of any of claims 6 to 9, further comprising one or more sensors configured to obtain a rotational velocity of the rotor.

11. The infusion device of any of the claims 6 to 10, wherein the rotor has a centerpoint that is radially offset from a central position defined by the stator, and/or
wherein the rotor has a centerpoint that is nearer to a first one of the plurality of coil windings than to a second one of the plurality of coil windings.

12. The infusion device of any of the claims 6 to 11, wherein the dispenser comprises a moveable plunger disposed within the reservoir, and wherein rotation of the rotor in the second direction causes the plunger to move within the reservoir to dispense medicament.

13. A device comprising:
a means for containing medicament;
a means for dispensing medicament from the medicament-containing means; and
a means for actuating the medicament-dispensing means, the actuating means comprising a rotor operably coupled to the medicament-dispensing means and configured to rotate the rotor in a first direction for a first rotational amount less than a full
rotation and to then rotate in a second, opposite direction for a second rotational amount more than the first rotational amount, wherein rotation in the second direction causes medicament to be dispensed from the medicament-containing means.

14. The device of claim 13, further comprising a means for sensing rotation of the rotor, wherein the device is configured to initiate rotation of the rotor in the second direction based on one or more signals from the rotation-sensing means, and/or
wherein the medicament-containing means comprises a reservoir, and/or
wherein the medicament-dispensing means comprises a plunger.

15. The device of claim 13 or 14, wherein the actuating means comprises an electromagnetic motor comprising a stator having a plurality of coil windings surrounding a central opening, the rotor comprising a permanent magnet removably disposed within the central opening.
